# EUROPEAN PATENT APPLICATION

(11) **EP 0 982 031 A2**
(43) Date of publication of application: **01.03.2000**
(21) Application number: 99306256.1
(22) Date of filing: 06.08.1999
(51) Int. Cl.: A61K 31/506, A61K 31/4375

(54) **Antifungal compositions comprising voriconazole and trovafloxacin or prodrugs thereof**

(30) Priority: 21.08.1998 GB 9818258
(71) Applicant: PFIZER INC., New York, N.Y. 10017 (US); Pfizer Limited, Sandwich Kent CT13 9NJ (GB)
(72) Inventor: Bell, Andrew Simon, Pfizer Central Research, Sandwich, Kent CT13 9NJ (GB); Hitchcock, Christopher Alan, Pfizer Central Res., Sandwich, Kent CT13 9NJ (GB); Dorr, Patrick Karl, Pfizer Central Research, Sandwich, Kent CT13 9NJ (GB)
(74) Representative: Simpson, Alison Elizabeth Fraser

(57) **Abstract**

This invention relates to a pharmaceutical composition comprising voriconazole and trovafloxacin, or salts/prodrugs thereof such as alatrofloxacin, and to the use of such a composition for the treatment of a fungal disease or rhinosinusitis. The present invention also relates to a method of treatment of a fungal disease or rhinosinusitis using a synergistic combination of voriconazole and trovafloxacin, or salts/prodrugs thereof such as alatrofloxacin.

## Description

This invention relates to antifungal compositions. More particularly, this invention relates to a pharmaceutical composition comprising voriconazole and trovafloxacin, or salts/prodrugs thereof such as alatrofloxacin, and to the use of such a composition for the treatment of a fungal disease or rhinosinusitis. The present invention also relates to a method of treatment of a fungal disease or rhinosinusitis using a synergistic combination of voriconazole and trovafloxacin, or salts/prodrugs thereof such as alatrofloxacin.

Voriconazole, 2R,3S-2-(2,4-difluorophenyl)-3-(5-fluoropyrimidin-4-yl)-1-(1H-1,2,4-triazol-1-yl)butan-2-ol, and pharmaceutically acceptable salts thereof, are disclosed in EP-A-0440372 as antifungal agents. Voriconazole is also generally disclosed in EP-A-0357241. Pharmaceutical formulations comprising voriconazole and a cyclodextrin derivative are described in WO 98/58677.

Trovafloxacin, (1-alpha, 5-alpha, 6-alpha)-7-(6-amino-3-azabicyclo[3.1.0]hex-3-yl-1-(2,4-difluorophenyl)-6-fluoro-1,4-dihydro-4-oxo-1,8-naphthyridine-3-carboxylic acid, and pharmaceutically acceptable salts thereof, and alatrofloxacin, (1-alpha, 5-alpha, 6-alpha)-L-alanyl-N-[3-[6-carboxy-8-(2,4-difluorophenyl )-3-fluoro-5,8-dihydro-5-oxo-1,8-naphthyridin-2-yl]-3-azabicyclo[3.1.0]hex-6-yl]-L-alaninamide, and pharmaceutically acceptable salts thereof, are disclosed in EP-A-0413455 as antibacterial agents with a broad spectrum of activity, particularly for the treatment of gram-positive bacterial strains. An anhydrous, crystalline form of the methanesulphonate salt of trovafloxacin is disclosed in EP-A-0789697. Alatrofloxacin is a prodrug form of trovafloxacin.

Sugar *et al,* Antimicrobial Agents and Chemotherapy, **41**(11), 2518-2521 (1997), reported that trovafloxacin was inactive *in vitro* against a variety of fungi. These authors also reported that a combination of fluconazole and trovafloxacin was more effective *in vivo* in prolonging the survival of mice infected with *Candida albicans* than was fluconazole alone, although the mechanism of action was not established.

It has also been reported that there may be a link between rhinosinusitis and fungal infections.

It has now been surprisingly found that trovafloxacin and/or alatrofloxacin enhance(s) the antifungal activity of voriconazole, and the activity of voriconazole in treating rhinosinusitis, in a synergistic manner.

Accordingly, the present invention provides a pharmaceutical composition comprising
(a) voriconazole, or a pharmaceutically acceptable salt or prodrug thereof;
(b) trovafloxacin, or a pharmaceutically acceptable salt or prodrug thereof; and, optionally,
(c) a pharmaceutically acceptable diluent, excipient or carrier.

The present invention provides such a pharmaceutical composition for use as a medicament.

The present invention provides the use of such a pharmaceutical composition for the manufacture of an antifungal agent.

The present invention provides the use of such a pharmaceutical composition for the manufacture of a medicament for treating rhinosinusitis.

The present invention provides a method of treatment of a mammal, particularly a human being, to treat a fungal disease comprising administering to said mammal an effective amount of such a pharmaceutical composition.

The present invention provides a method of treatment of a mammal, particularly a human being, to treat rhinosinusitis comprising administering to said mammal an effective amount of such a pharmaceutical composition.

The present invention provides a method of treatment of a mammal, particularly a human being, to treat a fungal disease comprising administering to said mammal, simultaneously, separately or sequentially, synergistically effective amounts of voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof.

The present invention provides a method of treatment of a mammal, particularly a human being, to treat rhinosinusitis comprising administering to said mammal, simultaneously, separately or sequentially, synergistically effective amounts of voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof.

The present invention provides a product containing voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof, as a combined preparation for simultaneous, separate or sequential use in treating a fungal disease.

The present invention provides a product containing voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof, as a combined preparation for simultaneous, separate or sequential use in treating rhinosinusitis.

Such products may contain instructions for drug administration to treat these particular diseases.

A preferred prodrug of trovafloxacin for use in the present invention is alatrofloxacin.

The pharmaceutically acceptable salts of voriconazole and trovafloxacin include the acid addition and the base salts thereof.

Suitable acid addition salts are formed from acids which form non-toxic salts and examples are the hydrochloride, hydrobromide, hydroiodide, sulphate, bisulphate, nitrate, phosphate, hydrogen phosphate, acetate, maleate, fumarate, lactate, tartrate, citrate, gluconate, succinate, saccharate, benzoate, methanesulphonate, ethanesulphonate, benzenesulphonate, p-toluenesulphonate and pamoate salts.

Suitable base salts are formed from bases which form non-toxic salts and examples are the sodium, potassium, aluminium, calcium, magnesium, zinc and diethanolamine salts.

For a review on suitable salts see Berge *et al,* J. Pharm. Sci., **66**, 1-19 (1977).

Preferred salts of trovafloxacin and alatrofloxacin include the methanesulphonate and hydrochloride salts with the methanesulphonate salts being especially preferred.

Also included within the scope of the present invention are the pharmaceutically acceptable solvates and polymorphs of voriconazole and trovafloxacin, and of the salts/prodrugs thereof such as alatrofloxacin.

Suitable solvates for use in the present invention include hydrates.

Radiolabelled derivatives of voriconazole and trovafloxacin, and of the salts/prodrugs thereof such as alatrofloxacin, are also included within the scope of this invention.

Suitable prodrugs of voriconazole include the phosphate prodrugs described in WO 97/28169.

Suitable prodrugs of trovafloxacin are described in EP-A-0413455.

Suitable methods for preparing voriconazole and salts thereof are as described in EP-A-0440372, EP-A-0357241 and WO 97/06160.

Suitable methods for preparing trovafloxacin and alatrofloxacin and salts thereof are as described in EP-A-0413455 and EP-A-0789697.

Voriconazole and trovafloxacin, or salts/prodrugs thereof such as alatrofloxacin, can be administered alone but will generally be administered in admixture with a suitable pharmaceutical excipient, diluent or carrier selected with regard to the intended route of administration and standard pharmaceutical practice. Voriconazole and trovafloxacin, or salts/prodrugs thereof such as alatrofloxacin, may be administered simultaneously (e.g. as a combined preparation), separately or sequentially.

For example, voriconazole and trovafloxacin, or salts/prodrugs thereof such as alatrofloxacin, can be administered orally or sublingually in the form of tablets, capsules, ovules, elixirs, solutions or suspensions, which may contain flavouring or colouring agents, for immediate or controlled release applications.

Such tablets may contain excipients such as microcrystalline cellulose, lactose, sodium citrate, calcium carbonate, dicalcium phosphate and glycine, disintegrants such as starch (preferably corn, potato or tapioca starch), alginic acid and certain complex silicates, and granulation binders such as polyvinylpyrrolidone, sucrose, gelatin and acacia. Additionally, lubricating agents such as magnesium stearate, sodium lauryl sulfate and talc may be included.

Solid compositions of a similar type may also be employed as fillers in gelatin capsules. Preferred excipients in this regard include lactose or milk sugar as well as high molecular weight polyethylene glycols. For aqueous suspensions and/or elixirs, voriconazole and trovafloxacin, or salts/prodrugs thereof such as alatrofloxacin, may be combined with various sweetening or flavouring agents, colouring matter or dyes, with emulsifying and/or suspending agents and with diluents such as water, ethanol, propylene glycol and glycerin, and combinations thereof.

Voriconazole and trovafloxacin, or salts/prodrugs thereof such as alatrofloxacin, can also be injected parenterally, for example, intravenously, intraperitoneally, intrathecally, intraventricularly, intrasternally, intracranially, intramuscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients or animals, voriconazole, or salts/prodrugs thereof, may be co-administered with trovafloxacin, or salts/prodrugs thereof such as alatrofloxacin, at a dose (of voriconazole) of from 0.1 to 50, mg/kg. For human patients, the preferred dose range is from 1 to 10, and most preferably from 3 to 5, mg/kg, from one to four times daily. For animals, the preferred dose range is from 1 to 20 mg/kg.

For oral and parenteral administration to human patients or animals, trovafloxacin, or salts/prodrugs thereof such as alatrofloxacin, may be co-administered with voriconazole at a dose (of trovafloxacin or alatrofloxacin) of from 1 to 100, preferably from 10 to 40, mg/kg, from one to four times daily.

The physician will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

Voriconazole and trovafloxacin, or salts/prodrugs thereof such as alatrofloxacin, can also be administered intranasally or by inhalation and may be conveniently delivered in the form of a dry powder inhaler or an aerosol spray presentation from a pressurised container or a nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark] or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container or nebuliser may contain a solution or suspension of voriconazole and trovafloxacin, or salts/prodrugs thereof such as alatrofloxacin, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of voriconazole and trovafloxacin, or salts/prodrugs thereof such as alatrofloxacin, and a suitable powder base such as lactose or starch.

Alternatively, voriconazole and trovafloxacin, or salts/prodrugs thereof such as alatrofloxacin, can be administered in the form of a suppository or pessary, or they may be applied topically in the form of a lotion, solution, cream, ointment or dusting powder. Voriconazole and trovafloxacin, or salts/prodrugs thereof such as alatrofloxacin, may also be transdermally administered by the use of a skin patch.

For ophthalmic use, voriconazole and trovafloxacin, or salts/prodrugs thereof such as alatrofloxacin, can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, voriconazole and trovafloxacin, or salts/prodrugs thereof such as alatrofloxacin, can be formulated as a suitable ointment containing voriconazole and trovafloxacin, or salts/prodrugs thereof such as alatrofloxacin, suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Voriconazole can also be formulated by complexation with a cyclodextrin. Particularly preferred for this purpose are the sulphoalkylether cyclodextrin derivatives of WO 91/11172, especially the sulphobutyl-substituted beta-cyclodextrin derivatives. The hydroxyalkyl cyclodextrin derivatives of EP-A-0149197 may also be used. A preferred voriconazole/cyclodextrin formulation is described in WO 98/58677.

The pharmaceutical composition or synergistic combination of voriconazole and trovafloxacin, or salts/prodrugs thereof such as alatrofloxacin, of the present invention may be used to treat fungal infections in mammals, including human beings. For example, they are useful in treating superficial fungal infections caused by, amongst other organisms, species of *Candida, Trichophyton, Microsporum* or *Epidermophyton,* or in treating mucosal infections caused by *Candida albicans* (e.g,. thrush and vaginal candidiasis). They can also be used in the treatment of systemic fungal infections caused by, for example, species of *Candida* (e.g. *Candida albicans), Cryptococcus* (e.g. *Cryptococcus neoformans),* Aspergillus (e.g. *Aspergillus flavus, Aspergillus fumigatus), Coccidioides, Paracoccidioides, Histoplasma* or *Blastomyces.*

The pharmaceutical composition or synergistic combination of voriconazole and trovafloxacin, or salts/prodrugs thereof such as alatrofloxacin, of the present invention may be used to treat rhinosinusitis, particularly chronic rhinosinusitis, of an invasive or non-invasive nature, in mammals, including human beings.

It is to be appreciated that all references herein to treatment include curative, palliative and prophylactic treatment.

### PHARMACOLOGICAL TESTING

The *in vivo* efficacy of voriconazole, trovafloxacin or alatrofloxacin, and of combinations thereof, against guinea pig sub-acute candidosis can be determined as follows.

Guinea pigs (Charles River SPF male, average weight 500g) (5 animals *per* group) are infected intravenously with an inoculum of *Candida albicans* (1x10⁶ cells *per* animal in 200 microlitres of 0.85% w/v of sterile saline solution). The test compound(s) (i.e. voriconazole, trovafloxacin and/or alatrofloxacin) are examined for efficacy against systemic infections in this animal model following oral administration (b.i.d) at various dose levels for 5 days. Efficacy is assessed by establishing the reduction in recoverable pathogen colon-forming units (c.f.u.) from harvested tissues (kidney and liver) of animals that have been dosed with the test compound(s) compared to those from untreated control animals that have been treated with the vehicle only. Increased survival rate for the treated *versus* the untreated infected animal group is also a measure of the efficacy of the test compound(s).

Suitable treatment groups for this *in vivo* study are:
1. Control (vehicle only - PEG 200) - b.i.d.
2. Voriconazole - 10mg/kg b.i.d.
3. Trovafloxacin or alatrofloxacin- 10mg/kg b.i.d.
4. Voriconazole/trovafloxacin or alatrofloxacin- both 10mg/kg b.i.d.

In the above *in vivo* protocol, all dosing is carried out by oral gavage at 0.5ml per dose commencing 1 hour post-infection and continued for 5 days. The animals are euthanased on Day 6, the livers and kidneys harvested, homogenised and the homogenate serially diluted with 0.85% w/v sterile saline and then plated onto Sabouraud agar plates containing 50 micrograms/ml of oxytetracycline. After 2 days incubation at 28 °C the fungal colonies are counted and the average log. c.f.u./g tissue established for each treatment group.

## Claims

1. A pharmaceutical composition comprising
(a) voriconazole, or a pharmaceutically acceptable salt or prodrug thereof;
(b) trovafloxacin, or a pharmaceutically acceptable salt or prodrug thereof; and, optionally,
(c) a pharmaceutically acceptable diluent, excipient or carrier.

2. A composition as claimed in claim 1 wherein the salt of trovafloxacin is a methanesulphonate salt.

3. A composition as claimed in claim 1 wherein the prodrug of trovafloxacin is alatrofloxacin.

4. A pharmaceutical composition as claimed in claim 1, 2 or 3 for use as a medicament.

5. The use of a pharmaceutical composition as claimed in claim 1, 2 or 3 for the manufacture of an antifungal agent.

6. The use of a pharmaceutical composition as claimed in claim 1, 2 or 3 for the manufacture of a medicament for the treatment of rhinosinusitis.

7. A method of treatment of a mammal, particularly a human being, to treat a fungal disease comprising administering to said mammal, simultaneously, separately or sequentially, synergistically effective amounts of voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof.

8. A method of treatment of a mammal, particularly a human being, to treat a fungal disease comprising administering to said mammal an effective amount of a composition as claimed in claim 1, 2 or 3.

9. A method of treatment of a mammal, particularly a human being, to treat rhinosinusitis comprising administering to said mammal, simultaneously, separately or sequentially, synergistically effective amounts of voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof.

10. A method of treatment of a mammal, particularly a human being, to treat rhinosinusitis comprising administering to said mammal an effective amount of a composition as claimed in claim 1, 2 or 3.

11. A method as claimed in claim 7, 8, 9 or 10 wherein the salt of trovafloxacin is a methanesulphonate salt.

12. A method as claimed in claim 7, 8, 9 or 10 wherein the prodrug of trovafloxacin is alatrofloxacin.

13. A product containing voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof, as a combined preparation for simultaneous, separate or sequential use in treating a fungal disease.

14. A product containing voriconazole, or a pharmaceutically acceptable salt or prodrug thereof, or a pharmaceutically acceptable composition thereof, and trovafloxacin, or a pharmaceutically acceptable salt or prodrug thereof, or a pharmaceutically acceptable composition thereof, as a combined preparation for simultaneous, separate or sequential use in treating rhinosinusitis.

15. A product as claimed in claim 13 or 14 wherein the salt of trovafloxacin is a methanesulphonate salt.

16. A product as claimed in claim 13 or 14 wherein the prodrug of trovafloxacin is alatrofloxacin.
